# EUROPEAN PATENT APPLICATION

(11) **EP 4 617 647 A1**
(43) Date of publication of application: **17.09.2025**
(21) Application number: 25164239.3
(22) Date of filing: 17.03.2025
(51) Int. Cl.: G01N 27/16, G01N 33/00

(54) **A HYDROGEN GAS SENSOR AND A METHOD OF FABRICATING THEREOF**

(30) Priority: 15.03.2024 US 202463565762 P
(71) Applicant: Detector Electronics Buyer US, LLC, Bloomington, MN 55438 (US); Carrier Corporation, Palm Beach Gardens, FL 33418 (US)
(72) Inventor: EVJU, Jon, Bloomington, 55438 (US); JARVIS, John, Bloomington, 55438 (US)
(74) Representative: Dehns

(57) **Abstract**

Described herein is a method for fabricating a hydrogen specific gas sensor. The method comprises the steps of: enabling chemical vapor deposition of siloxane or organosilane precursor gas mixture over a catalytic sensor at a predetermined temperature to deposit a layer of porous silica over active catalytic sites associated with the catalytic sensor, wherein the deposited porous silica allows hydrogen gas and/or oxygen to diffuse therethrough and interact with the active catalytic sites of the catalytic sensor, resulting in detection of the hydrogen gas.

## Description

### BACKGROUND

This invention relates to the field of combustible gas sensors, and more particularly, a hydrogen gas sensor and a method of fabricating thereof.

### SUMMARY

Described herein is a method for fabricating a hydrogen specific gas sensor. The method comprises the steps of enabling chemical vapor deposition of siloxane or organosilane precursor gas mixture over a catalytic sensor at a predetermined temperature to deposit a layer of porous silica over active catalytic sites associated with the catalytic sensor, wherein the deposited porous silica allows hydrogen gas and/or oxygen to diffuse therethrough and interact with the active catalytic sites of the catalytic sensor, resulting in detection of the hydrogen gas.

In one or more embodiments, the method comprises the step of maintaining the catalytic sensor at the predetermined temperature to further reduce catalytic interaction between the catalytic sensor and one or more combustible gases having molecular size greater than molecular size of hydrogen gas.

In one or more embodiments, the catalytic sensor is maintained at the predetermined temperature during the deposition of porous silica on the catalytic sensor and/or during the hydrogen gas sensing operation by the sensor.

In one or more embodiments, the method comprises the step of disposing a heating element with a catalytically active coating and a reference electrode without a catalytically active coating within, adjacent to, separated from, or in thermal contact with the catalytic sensor in a non-combustible gas atmosphere, wherein the heating element and a driving circuit are configured to maintain the catalytic sensor at the predetermined temperature or measure a difference in temperature, resistance, or difference in electrical power required to maintain the predetermined temperature in a combustible gas atmosphere.

In one or more embodiments, the method comprises the step of disposing the heating element having a predetermined resistance and a predetermined stiffness in a predefined coil arrangement within the catalytic sensor to support and strengthen the catalytic sensor, while heating or maintaining the catalytic sensor at the predetermined temperature.

In one or more embodiments, the method of fabricating the catalytic sensor comprises the step of: disposing the heating element and the reference electrode within a substrate; depositing a first layer of an inert refractive material on the substrate; and coating a second layer of a catalyst on the first layer to fabricate the catalytic sensor having the active catalytic sites.

In one or more embodiments, the method further comprises the step of coating a third layer of a nanoporous glassy or ceramic material on the catalytic sensor having the deposited porous silica thereon, wherein the third layer allows the hydrogen gas and/or the oxygen to diffuse therethrough and interact with the active catalytic sites of the catalytic sensor.

In one or more embodiments, the heating element is made of a material selected from a group of refractory materials and alloys comprising gold, pure platinum metal, platinum-based alloy, and platinum iridium alloy.

In one or more embodiments, the catalytic sensor or the catalyst is selected from a group of materials comprising platinum, and palladium, and wherein the siloxane precursor gas is hexamethyldisiloxane.

Also described herein is a hydrogen gas sensor. The hydrogen gas sensor comprises a catalytic gas sensor, and a layer of porous silica deposited over active catalytic sites associated with the catalytic sensor, wherein the deposited porous silica allows hydrogen gas and/or oxygen to diffuse and interact with the active catalytic sites of the catalytic sensor, resulting in detection of the hydrogen gas.

In one or more embodiments, the layer of porous silica is deposited over the catalytic sensor by chemical vapor deposition of siloxane precursor gas at a predetermined temperature over the catalytic sensor.

In one or more embodiments, the catalytic sensor is maintained at the predetermined temperature to reduce catalytic interaction between the catalytic sensor and one or more combustible gases having molecular size greater than molecular size of hydrogen gas.

In one or more embodiments, the catalytic sensor is maintained at the predetermined temperature during the deposition of porous silica on the catalytic sensor and/or during the hydrogen gas sensing operation by the sensor.

In one or more embodiments, temperature of the catalytic sensor is lowered below the predetermined temperature to increase catalytic interaction between the catalytic sensor and the precursor gas, and wherein temperature of the catalytic sensor is increased above the predetermined temperature to remove byproducts formed on surface of the catalytic sensor upon breaking of the precursor gas into a glass form.

In one or more embodiments, the sensor comprises a heating element and a reference electrode disposed within or in thermal contact with the catalytic sensor, wherein the heating element is configured to heat and/or maintain the catalytic sensor at the predetermined temperature.

In one or more embodiments, the heating element having a predetermined resistance and a predetermined stiffness is disposed within the catalytic sensor in a predefined coil arrangement to support and strengthen the catalytic sensor.

In one or more embodiments, the catalytic sensor comprises a substrate, a first layer of an inert refractory material coated on the substrate, and a second layer of a catalyst coated on the first layer to fabricate the catalytic sensor having the active catalytic sites, wherein the heating element and the reference electrode are disposed within the substrate.

In one or more embodiments, the sensor further comprises a third layer of a nanoporous glassy or ceramic material coated on the catalytic sensor having the deposited porous silica thereon, wherein the third layer allows the hydrogen gas and/or the oxygen to diffuse therethrough and interact with the active catalytic sites of the catalytic sensor.

In one or more embodiments, the heating element is made of a material selected from a group comprising gold, pure platinum metal, platinum-based alloy, and platinum iridium alloy.

In one or more embodiments, the catalytic sensor or the catalyst is selected from a group of materials comprising platinum, and palladium, and wherein the siloxane precursor gas is hexamethyldisiloxane.

In one or more embodiments, the sensor is operated in any of a constant current mode, a constant power mode, a constant voltage mode, and/or a constant temperature mode to determine and monitor a concentration of the detected hydrogen gas based on the interaction between the hydrogen gas and the active catalytic sites.

The hydrogen gas sensor may be formed by the method for fabricating a hydrogen specific gas sensor described herein and optionally may be formed by any of the optional features described herein in relation to the method for fabricating a hydrogen specific gas sensor.

The foregoing summary is illustrative only and is not intended to be in any way limiting. In addition to the illustrative aspects, embodiments, and features described above, further aspects, embodiments, features, and techniques of the subject disclosure will become more apparent from the following description taken in conjunction with the drawings.

### BRIEF DESCRIPTION OF THE DRAWINGS

The accompanying drawings are included to provide a further understanding of the subject disclosure and are incorporated in and constitute a part of this specification. The drawings illustrate exemplary embodiments of the subject disclosure and, together with the description, serve to explain the principles of the subject disclosure.

In the drawings, similar components and/or features may have the same reference label. Further, various components of the same type may be distinguished by following the reference label with a second label that distinguishes among the similar components. If only the first reference label is used in the specification, the description is applicable to any one of the similar components having the same first reference label irrespective of the second reference label.
FIG. 1 illustrates an exemplary flow diagram depicting the method for fabricating a hydrogen gas sensor in accordance with one or more embodiments of the subject disclosure.
FIG. 2 illustrates an exemplary flow diagram depicting the method for fabricating the catalytic sensor for the hydrogen gas sensor in accordance with one or more embodiments of the subject disclosure.
FIG. 3 illustrates an exemplary representation of the hydrogen gas sensor depicting various layers of the sensor in accordance with one or more embodiments of the subject disclosure.

### DETAILED DESCRIPTION

The following is a detailed description of embodiments of the subject disclosure depicted in the accompanying drawings. The embodiments are in such detail as to clearly communicate the subject disclosure. However, the amount of detail offered is not intended to limit the anticipated variations of embodiments; on the contrary, the intention is to cover all modifications, equivalents, and alternatives falling within the spirit and scope of the subject disclosure as defined by the appended claims.

Various terms are used herein. To the extent a term used in a claim is not defined below, it should be given the broadest definition persons in the pertinent art have given that term as reflected in printed publications and issued patents at the time of filing.

In the specification, reference may be made to the spatial relationships between various components and to the spatial orientation of various aspects of components as the devices are depicted in the attached drawings. However, as will be recognized by those skilled in the art after a complete reading of the subject disclosure, the components of this invention. described herein may be positioned in any desired orientation. Thus, the use of terms such as "above," "below," "upper," "lower," "first", "second" or other like terms to describe a spatial relationship between various components or to describe the spatial orientation of aspects of such components should be understood to describe a relative relationship between the components or a spatial orientation of aspects of such components, respectively, described herein may be oriented in any desired direction.

In recent years, the growing prominence of the hydrogen economy has necessitated the development of advanced detection systems capable of precisely monitoring hydrogen levels at concentrations corresponding to fractions of the Lowest Flammable Level (LEL). As hydrogen emerges as a key player in various industries, including energy, transportation, and manufacturing, the need for robust, reliable, stable and selective hydrogen detectors becomes paramount. The current landscape of combustible gas sensors presents a notable challenge in meeting the specific requirements of hydrogen detection.

Existing sensors designed for combustible gases lack the requisite selectivity to discriminate between different gases accurately. This lack of specificity becomes a critical concern in applications where the detection of hydrogen is required without the risk of false alarms triggered by incidental exposure to small amounts of hydrocarbons or cleaning solutions.

Existing solutions used for combustible gas detection are not tailored to the unique characteristics of hydrogen. Combustible gas sensors typically respond to a broad spectrum of gases, making them susceptible to false positives and rendering them less reliable in scenarios where precise hydrogen detection is essential. The consequence of false alerts may lead to unnecessary disruptions, increased maintenance costs and compromised safety protocols.

There is therefore a need for a solution to effectively address the challenges of associated with existing combustible gas sensors, by providing a solution to selectively detect hydrogen gas, without the risk of false triggering by other large molecule combustible gases such as hydrocarbons or cleaning solutions.

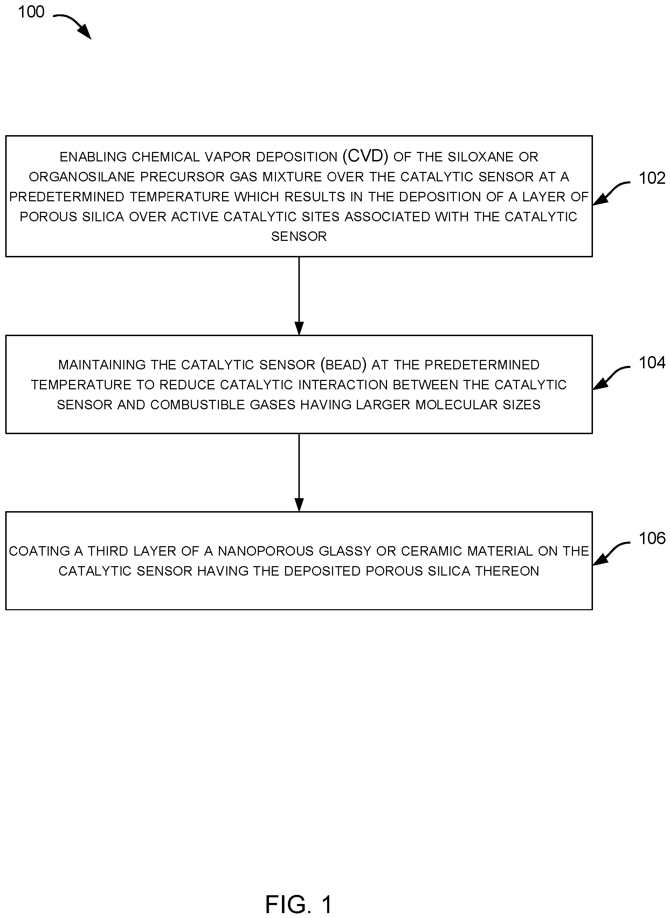

Referring to FIG. 1, a method 100 for fabricating a hydrogen gas sensor (also referred to as sensor, herein) is disclosed. In one or more embodiments, the method 100 may involve a catalytic sensor with active catalytic sites and a siloxane precursor gas. Further, a furnace or heating equipment may be used for chemical vapor deposition, a gas flow system may be used for introducing the siloxane precursor gases into the furnace, and a control system may be used for precise control of catalytic sensor (bead) temperature and electrical attributes (current, voltage, and power) associated with the sensor. It is to be appreciated that the various components involved in implementing method 100 described herein are only exemplary, and these may be changed without any limitation whatsoever, and all such implementations are well within the scope of the present application.

In one or more embodiments, the method 100 may include step 102 of enabling chemical vapor deposition (CVD) of the siloxane or organosilane precursor gas mixtures over the catalytic sensor at a predetermined temperature which results in the deposition of a layer of porous silica over active catalytic sites associated with the catalytic sensor. The deposited porous silica may allow hydrogen gas and/or oxygen to diffuse therethrough and interact with the active catalytic sites of the catalytic sensor, resulting in the detection of the hydrogen gas. However, the deposited porous silica may "poison" the catalytic sensor against large molecule combustible gases (having a size greater than the size of hydrogen gas and oxygen), by restricting or blocking such large molecule combustible gases from passing therethrough. These large molecule combustible gases may comprise any combustible gas or solvent vapors such as but not limited to ethanol, methane, propane, ethanol, toluene, olefins, and cleaning solutions.

In one or more embodiments, the CVD of the siloxane precursor gas over the catalytic sensor may be performed in a furnace chamber, however, the CVD may also be performed in a non-confined environment at atmospheric pressure using the heating equipment. At step 102, the method may include placing the catalytic sensor inside the furnace, which may be preheated to an elevated temperature using the heating equipment. Further, the siloxane precursor gas may be introduced into the furnace using the gas flow system and the catalytic sensor may be heated at the predetermined temperature which may be in a range of 500 to 700 °C. Accordingly, the siloxane precursor gas may undergo CVD and deposit porous silica on the hot active catalytic sites of the sensor. This process may thus create small pores on the sensor surface, that provide a barrier to larger molecules by physically blocking their access to the catalytic sites.

In one or more embodiments, the method may include step 104 of maintaining the catalytic sensor (bead) at the predetermined temperature to reduce catalytic interaction between the catalytic sensor and combustible gases having larger molecular sizes. The catalytic sensor may be maintained at the predetermined temperature during the deposition of porous silica on the catalytic sensor as well as during the hydrogen gas sensing operation by the sensor. However, the temperature of the catalytic sensor may also be kept lower or higher than the predetermined temperature during the hydrogen gas sensing operation. In one or more embodiments, the temperature of the catalytic sensor may be lowered below the predetermined temperature to increase catalytic interaction between the catalytic sensor and the precursor gas. Further, the temperature of the catalytic sensor may be increased above the predetermined temperature to remove byproducts formed on the surface of the catalytic sensor upon breaking of the precursor gas into a glass form

To enhance the sensor's selectivity towards hydrogen, the temperature of the catalytic sensor (bead) may be lowered during sensing operation to reduce the catalytic activity of the catalyst toward other combustible gases and preferentially allowing oxidation of hydrogen gas over the catalytic sites. When hydrogen is introduced to the sensor, hydrogen may diffuse rapidly through the porous silica and react with the catalytic sites, causing an exothermic reaction at the catalytic sensor surface, changing the temperature and electrical resistivity, and thermal properties of the catalytic sensor. This change in electrical and/or thermal conductivity may be measured and used as an output signal, which may be further calibrated using a reference electrode (also referred to as a reference bead) to provide a quantitative measurement of the hydrogen concentration. In addition, the sensor is continuously regenerated after saturation upon interaction of the hydrogen gas (captured in silica pores) with oxygen present around the sensor, without the need of any additional regeneration systems or process.

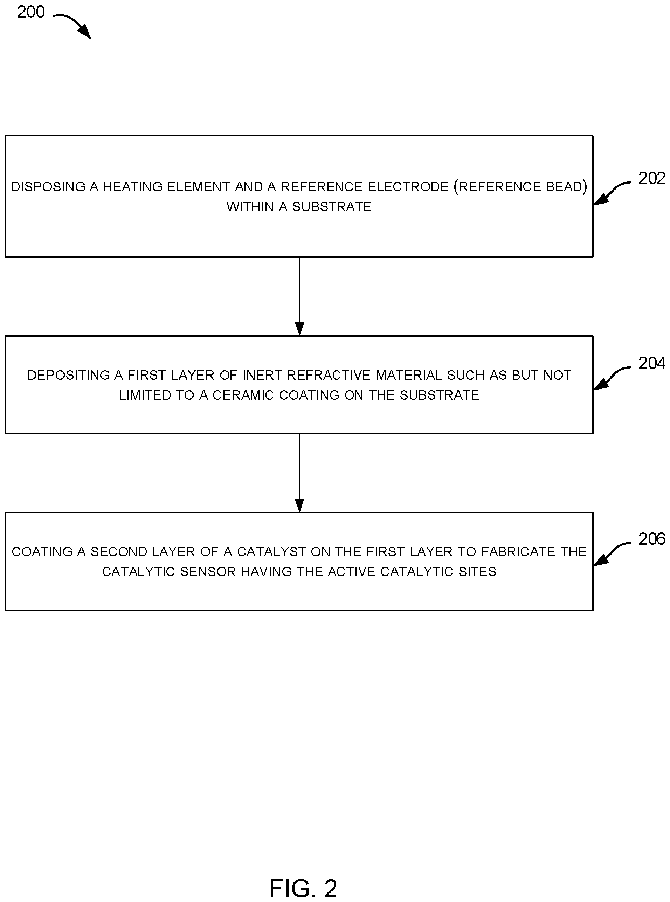

Referring to FIG. 2, in one or more embodiments, a method 200 for fabricating the catalytic sensor is disclosed. The method 200 may include step 202 of disposing a heating element (also referred to as detector bead or active bead) and the reference electrode (reference bead) within a substrate. Further, method 200 may include step 204 of depositing a first layer of inert refractive material such as but not limited to a ceramic coating on the substrate, followed by another step 206 of coating a second layer of a catalyst on the first layer to fabricate the catalytic sensor having the active catalytic sites. The additional layers may intentionally contain materials that may increase the surface area of the sensor.

In one or more embodiments, step 202 may include disposing the heating element having a predetermined electrical resistance and a predetermined stiffness in a predefined coil arrangement within the catalytic sensor or substrate to support and strengthen the catalytic sensor. The heating element may further be enabled at constant heating or maintaining the catalytic sensor at the predetermined temperature during sensor fabrication as well as for lowering the bead temperature during the hydrogen sensing operation. However, in some embodiments, the heating element and the reference bead may not be disposed within the catalytic sensor but may be just in thermal contact with the catalytic sensor, where the heating element is configured to heat and/or maintain the catalytic sensor at the predetermined temperature and may further enable measurement of the temperature of the catalytic sensor or bead temperature to facilitate in computing the sensed hydrogen gas concentration.

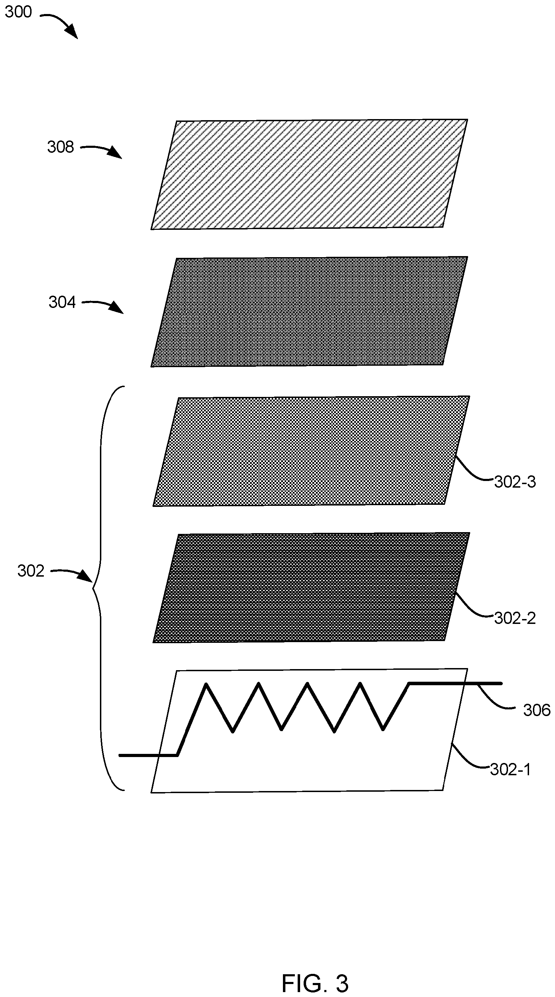

In one or more embodiments, the substrate may be a ceramic substrate. Further, the substrate may also be a MEMS-based substrate that may provide distinct advantages over the traditional handmade, hand-painted ceramic substrate having a lower thermal conductivity. The MEMS-based substrate may be made of silicon, Si3N4 (silicon nitride), SiC (silicon carbide), beryllium oxide, quartz, ceramics (e.g. steatite, porcelain, alumina), aluminum nitride, silica, sapphire, or other suitable material that is itself thermally conductive, or includes an embedded lateral heat transfer device (heating element 306 of FIG. 3), in order to enable the heater to more uniformly, consistently, and quickly heat the substrate. In addition, the substrate may also be embedded with a heat sensor along with the heating element.

Referring back to FIG. 1, the method 100 for fabricating the sensor may additionally include step 106 of coating an additional (or third) layer of a nanoporous glassy or ceramic material on the catalytic sensor having the deposited porous silica thereon. However, there may be various other layers (containing catalysts and other materials) underneath the third layer. The third layer may allow the hydrogen gas and/or the oxygen to diffuse therethrough and interact with the active catalytic sites of the catalytic sensor.

Referring to FIG. 3, an exemplary representation depicting various layers of the hydrogen gas sensor 300 is disclosed. The sensor 300 may include a catalytic sensor 302, and a layer of porous silica 304 deposited over active catalytic sites associated with the catalytic sensor 302. The deposited porous silica 304 may allow hydrogen gas and/or oxygen to diffuse therethrough and interact with the active catalytic sites of the catalytic sensor 302, resulting in the detection of the hydrogen gas. However, the deposited porous silica 304 may "poison" the catalytic sensor 302 against large molecule combustible gases (having a size greater than the size of hydrogen gas and oxygen), by restricting or blocking such large molecule combustible gases from passing therethrough.

In one or more embodiments, the layer of porous silica 304 may be deposited over the catalytic sensor 302 by chemical vapor deposition (CVD) of siloxane precursor gas at a predetermined temperature (130 to 600 °C) over the catalytic sensor 302. In addition, the catalytic sensor 302 may be maintained at the predetermined temperature to reduce catalytic interaction between the catalytic sensor and the combustible gases having larger molecular sizes. However, the temperature of the catalytic sensor may also be kept lower or higher than the predetermined temperature.

The catalytic sensor 302 may include a heating element (detector bead) 306 and a reference electrode (or reference bead or reference element) (not shown) disposed within or in thermal contact with the catalytic sensor 302. In one or more embodiments, the heating element 306 may have a predetermined resistance and a predetermined stiffness, which may be disposed within the catalytic sensor 302 in a predefined coil arrangement to support and strengthen the catalytic sensor 302, while enabling heating and/or maintain the catalytic sensor 302 at the predetermined temperature and further enabling measurement of the bead temperature of the catalytic sensor 302 to compute the sensed hydrogen gas concentration. In one or more embodiments, the heating element 306 with a catalytically active coating and the reference element without catalytically active coating within, adjacent to, separated from, or in contact with the catalytic sensor 302 in a non-combustible gas atmosphere. Further, the heating element 306 and a driving circuit may be configured to maintain the catalytic sensor 302 at the predetermined temperature or measure a difference in temperature, resistance, or difference in electrical power required to maintain the predetermined temperature in a combustible gas atmosphere.

In one or more embodiments, the catalytic sensor 302 or sensor 300 may include a substrate 302-1, a first layer 302-2 of an inert refractive material coated on the substrate 302-1, and a second layer 302-3 of a catalyst coated on the first layer 302-2 to fabricate the catalytic sensor 302 having the active catalytic sites. Further, the heating element (detector bead) 306 and the reference electrode (reference bead) may also be disposed within the substrate 302-1 during fabrication of the catalytic sensor 302. In addition, the sensor 300 may further include a third layer 308 of a nanoporous glassy or ceramic material coated on the catalytic sensor having the deposited porous silica thereon. However, there may be additional layers between the third layer 308 and the second layer 302-3. This third layer 308 may allow the hydrogen gas and/or the oxygen to diffuse therethrough and interact with the active catalytic sites of the catalytic sensor 302 while restricting or blocking large molecule combustible gases having a size greater than the size of hydrogen gas to pass therethrough. It is to be appreciated by a person skilled in the art that the porous silica layer deposited over the catalytic sensor mitigates the risk of false triggering of the sensor by other large molecule combustible gases, thereby making the sensor reliable and effective in hydrogen sensing.

In one or more embodiments, the heating element 306 may be made of a material selected from a group comprising gold, pure platinum metal, platinum-based alloy, and platinum-iridium alloy, but is not limited to the like. For example, when pure platinum is implemented as the heating element, the heating element 306 may have the predetermined electrical resistance of 10.5 microohm-cm and a predetermined stiffness of 41.52 N/m. This may provide support and strength to the catalytic sensor 302 and also enable heating of the catalytic sensor 302. Further, the catalytic sensor or the catalyst for the substrate 302-1 may be selected from a group of materials comprising platinum, palladium, and the like. Furthermore, the siloxane precursor gas may be hexamethyldisiloxane, but is not limited to the like.

Moreover, for further enhancing the sensor's 300 selectivity towards hydrogen, the temperature of the catalytic sensor (bead) 302 may be lowered to a range of 300 °C to 500 °C during sensing operation to reduce the catalytic activity of the catalyst toward other combustible gases while preferentially allowing oxidation of hydrogen gas at the catalytic sites. Accordingly, when hydrogen is introduced to the sensor 300, hydrogen may diffuse rapidly through the porous silica and react with the catalytic sites, causing a change in the electrical and thermal properties of the catalytic sensor 302. This change in electrical and/or thermal conductivity may be measured and used as an output signal, which may be further calibrated using the inactive reference sensor to provide a quantitative measurement of the hydrogen concentration.

In one or more embodiments, the fabricated sensor 300 may be operated in any of a constant current mode, a constant power mode, a constant voltage mode, and/or a constant temperature mode by a control system to determine and monitor the concentration of the sensed hydrogen gas based on the interaction between the hydrogen gas and the active catalytic sites.

In an example, in the constant current mode, the control system may enable a power source to pass constant electrical current through the heating element that may heat up the catalytic sensor to approximately 500 to 600 °C or above. At this temperature the active catalytic sites of the catalytic sensor may burn the target hydrogen gas being diffused through the pores of the deposited porous silica, thereby producing heat. The resulting temperature rise may be further detected by the control system using the heating element (whose resistance rises), as the resistance of the heating element is proportional to temperature. As the sensor is operated by maintaining a constant current through the heating element, the control system adjusts the applied current to ensure a consistent and stable temperature of the catalytic element. The amount of heat generated by the catalytic reaction is proportional to the adjusted current which may be further proportional to the concentration of the detected hydrogen gas. By keeping the current constant, the temperature of the catalytic element is also kept constant, providing a reliable and linear relationship between the generated heat and the sensed hydrogen gas concentration.

In another example, in the constant voltage mode, the control system may enable the power source to supply constant electrical voltage across the heating element that may heat up the catalytic sensor, however, the temperature of the catalytic sensor generally remains lower compared to the constant current mode. Similarly, at this temperature the active catalytic sites of the catalytic sensor may burn the target hydrogen gas being diffused through the pores of the deposited porous silica, thereby producing heat. The resulting temperature rise may be further detected by the control system using the heating element (whose resistance rises), as the resistance of the heating element is proportional to temperature. Similar to the constant current mode, the control system maintains a consistent temperature for the catalytic reaction, where the voltage is adjusted based on the resistance changes of the heated element in response to the catalytic reaction, thereby determining the sensed hydrogen gas concentration.

In yet another example, in the constant temperature mode, the control system may operate the sensor by maintaining a constant temperature on the catalytic sensor, regardless of changes in resistance or current. The control system includes a resistance/temperature feedback loop comprising a calorimeter and the like that may monitor the temperature of the catalytic sensor and adjust the current or voltage to maintain the desired temperature in the catalytic sensor. Accordingly, the control system maintains a consistent temperature for the catalytic reaction, where the voltage and/or the current are adjusted based on the resistance changes of the heated element in response to the catalytic reaction, thereby determining the sensed hydrogen gas concentration.

Further, in one or more embodiments, in order to compensate for environmental factors such as temperature and pressure, the active detector bead (heating element) may be matched with the reference bead. This pair of beads may generally run in a simple Wheatstone Bridge circuit.

Thus, this invention overcomes the challenges associated with existing combustible gas sensors, by providing a combustible gas sensor that selectively detects hydrogen gas, without the risk of false triggering by other large molecule combustible gases such as hydrocarbons or cleaning solutions.

While the subject disclosure has been described with reference to exemplary embodiments, it will be understood by those skilled in the art that various changes may be made and equivalents may be substituted for elements thereof without departing from the scope of the subject disclosure as defined by the appended claims. Modifications may be made to adopt a particular situation or material to the teachings of the subject disclosure without departing from the scope thereof. Therefore, it is intended that the subject disclosure not be limited to the particular embodiment disclosed, but that the subject disclosure includes all embodiments falling within the scope of the subject disclosure as defined by the appended claims.

In interpreting the specification, all terms should be interpreted in the broadest possible manner consistent with the context. In particular, the terms "comprises" and "comprising" should be interpreted as referring to elements, components, or steps in a non-exclusive manner, indicating that the referenced elements, components, or steps may be present, or utilized, or combined with other elements, components, or steps that are not expressly referenced. Where the specification claims refer to at least one of something selected from the group consisting of A, B, C ....and N, the text should be interpreted as requiring only one element from the group, not A plus N, or B plus N, etc.

## Claims

1. A method for fabricating a hydrogen specific gas sensor, the method comprising the steps of:
enabling chemical vapor deposition of siloxane or organosilane precursor gas mixture over a catalytic sensor at a predetermined temperature to deposit a layer of porous silica over active catalytic sites associated with the catalytic sensor,
wherein the deposited porous silica allows hydrogen gas and/or oxygen to diffuse therethrough and interact with the active catalytic sites of the catalytic sensor, resulting in detection of the hydrogen gas.

2. The method of claim 1, wherein the method comprises the step of maintaining the catalytic sensor at the predetermined temperature to further reduce catalytic interaction between the catalytic sensor and one or more combustible gases having molecular size greater than molecular size of hydrogen gas, and
wherein the catalytic sensor is optionally maintained at the predetermined temperature during the deposition of porous silica on the catalytic sensor and/or during the hydrogen gas sensing operation by the sensor.

3. The method of claims 1 or 2, wherein the method comprises the step of disposing a heating element with a catalytically active coating and a reference electrode without a catalytically active coating within, adjacent to, separated from, or in thermal contact with the catalytic sensor in a non-combustible gas atmosphere, wherein the heating element is configured to maintain the catalytic pellistor at the predetermined temperature and/or measure a difference in temperature, resistance, or difference in electrical power required to maintain the predetermined temperature in a combustible gas atmosphere.

4. The method of any one of claims 1 to 3, wherein the method comprises the step of disposing the heating element having a predetermined resistance and a predetermined stiffness in a predefined coil arrangement within the catalytic sensor to support and strengthen the catalytic sensor, while heating or maintaining the catalytic sensor at the predetermined temperature.

5. The method of any one of claims 1 to 4, wherein the method of fabricating the catalytic sensor comprises the steps of:
disposing the heating element and the reference electrode within a substrate;
depositing a first layer of an inert refractive material on the substrate; and
coating a second layer of a catalyst on the first layer to fabricate the catalytic sensor having the active catalytic sites, and
the method optionally further comprises the step of coating a third layer of a nanoporous glassy or ceramic material on the catalytic sensor having the deposited porous silica thereon, wherein the third layer allows the hydrogen gas and/or the oxygen to diffuse therethrough and interact at the active catalytic sites of the catalytic sensor.

6. The method of any one of claims 1 to 5, wherein: the heating element is made of a material selected from a group of refractory materials and alloys comprising gold, pure platinum metal, platinum-based alloy, and platinum iridium alloy; and/or
the catalytic sensor or the catalyst is selected from a group of material comprising platinum, and palladium, and wherein the siloxane precursor gas is hexamethyldisiloxane.

7. A hydrogen gas sensor comprising:
a catalytic sensor; and
a layer of porous silica deposited over active catalytic sites associated with the catalytic sensor,
wherein the deposited porous silica allows hydrogen gas and/or oxygen to diffuse and interact with the active catalytic sites of the catalytic sensor, resulting in detection of the hydrogen gas.

8. The hydrogen gas sensor of claim 7, wherein the layer of porous silica is deposited over the catalytic sensor by chemical vapor deposition of siloxane precursor gas at a predetermined temperature over the catalytic sensor.

9. The hydrogen gas sensor of any one of claims 7 and 8, wherein the catalytic sensor is maintained at the predetermined temperature to reduce catalytic interaction between the catalytic sensor and one or more combustible gases having molecular size greater than molecular size of hydrogen gas.

10. The hydrogen gas sensor of any one of claims 7 to 9, wherein temperature of the catalytic sensor is lowered below the predetermined temperature to increase catalytic interaction between the catalytic sensor and the precursor gas, and wherein temperature of the catalytic sensor is increased above the predetermined temperature to remove byproducts formed on surface of the catalytic sensor upon breaking of the precursor gas into a glass form, and
wherein the catalytic sensor is optionally maintained at the predetermined temperature during the deposition of porous silica on the catalytic sensor and/or during the hydrogen gas sensing operation by the sensor.

11. The hydrogen gas sensor of any one of claims 7 to 10, wherein the sensor comprises a heating element and a reference electrode disposed within or in thermal contact with the catalytic sensor, wherein the heating element is configured to heat and/or maintain the catalytic sensor at the predetermined temperature.

12. The hydrogen gas sensor of any one of claims 7 to 11, wherein the heating element having a predetermined resistance and a predetermined stiffness is disposed within the catalytic sensor in a predefined coil arrangement to support and strengthen the catalytic sensor.

13. The hydrogen gas sensor of any one of claims 7 to 12, wherein the catalytic sensor comprises:
a substrate;
a first layer of an inert refractory material coated on the substrate; and
a second layer of a catalyst coated on the first layer to fabricate the catalytic sensor having the active catalytic sites,
wherein the heating element and the reference electrode are disposed within the substrate, and
wherein the sensor optionally further comprises a third layer of a nanoporous glassy or ceramic material coated on the catalytic sensor having the deposited porous silica thereon, wherein the third layer allows the hydrogen gas and/or the oxygen to diffuse therethrough and interact with the active catalytic sites of the catalytic sensor.

14. The hydrogen gas sensor of any one of claims 7 to 13, wherein the heating element is made of a material selected from a group comprising gold, pure platinum metal, platinum-based alloy, and platinum iridium alloy.

15. The hydrogen gas sensor of any one of claims 7 to 14, wherein the catalytic sensor or the catalyst is selected from a group of materials comprising platinum, and palladium, and wherein the siloxane precursor gas is hexamethyldisiloxane, and/or wherein the hydrogen gas sensor is operated in any of a constant current mode, a constant power mode, a constant voltage mode, and/or a constant temperature mode to determine and monitor a concentration of the detected hydrogen gas based on the interaction between the hydrogen gas and the active catalytic sites.
